# EUROPEAN PATENT SPECIFICATION

(11) **EP 2 397 180 B1**
(45) Date of publication and mention of the grant of the patent: **22.01.2014**
(21) Application number: 10166531.3
(22) Date of filing: 18.06.2010
(51) Int. Cl.: A61M 25/02

(54) **Infusion site interface with improved tubing connection**
Infusionsstellenschnittstelle mit verbesserter Schlauchverbindung
Interface de site à perfusion avec connexion de tubage améliorée

(43) Date of publication of application: 21.12.2011
(73) Proprietor: F. Hoffmann-La Roche AG, 4070 Basel (CH); Roche Diagnostics GmbH, 68305 Mannheim (DE)
(72) Inventor: Pryor, Neil, Willingham CB24 5GD Cambridgeshire (GB); Pearson, Allen, Wisow Huntington PE28 2QB Cambridgeshire (GB); Collins, James, Yaxley Peterbourough PE7 3LW Cambridgeshire (GB)
(74) Representative: Rentsch Partner AG

(56) References cited:
- EP-A1- 1 820 525
- WO-A1-02/07804
- WO-A1-2008/092782

## Description

### Field of the Invention

The invention relates infusion site interfaces with soft infusion cannulas, particularly to infusion site interfaces used with infusion pump devices.

### State of the art

Devices for the automated release of liquid medicaments are generally used with patients who have a continuous but varying need of a medicine that can be administered by subcutaneous infusion. Specific applications are for example certain pain therapies and the treatment of diabetes. Infusion pump devices are particularly suitable for self-administration of liquid medicaments. In such cases, computer controlled infusion pump devices are used, which can be carried by the patient on the body, and which contain a certain amount of liquid medicament in a medicine reservoir. The medicine reservoir often comprises medicine sufficient for one to several days. The liquid medicament is supplied to the patient's body from the medicine reservoir through an infusion cannula or an injection needle, which is commonly placed in the subcutaneous body tissue.

A common type of infusion pump systems for continuous subcutaneous infusion comprises an infusion pump device fluidly connected to an infusion set, having an infusion site interface and infusion tubing. The infusion pump device is attached to a convenient place on the patient's body or his clothes, and the infusion tubing is fluidly connected to the pump device with a suitable connector. The infusion site interface is placed at a suitable position on the body, often in the abdominal region around and below the navel. Other possible infusion sites include upper leg, upper buttocks, hips, upper arms and lower back. The infusion site interface contains an infusion cannula, which is inserted into the subcutaneous tissue of the patient. To avoid medical problems such as infections, the infusion site interface has to be regularly replaced, usually after a few days.

The infusion cannula can be realized as a rigid or semi-rigid hollow needle, or as a soft, flexible cannula, for example in the form of a PTFE cannula. Soft cannulas are generally preferred. Soft infusion cannulas are well known in the prior art, for both intravenous and subcutaneous infusion. A rigid insertion needle arranged inside the soft cannula facilitates penetration of the skin of the patient and insertion of the cannula into the body tissue. After the infusion cannula has been placed, the insertion needle is removed from the cannula, while the soft cannula remains in the body tissue.

The infusion cannula can be inserted perpendicular to the body surface, or at an angle. Infusion site interfaces with angled cannulas have the advantage that the needle may be easier manually inserted in the body tissue, using the same technique as for standard injection needles. Furthermore the part of the cannula placed in the body can be longer for obtaining a certain infusion depth, which lowers the risk of the cannula accidentally leaving the body tissue during movements of the patient.

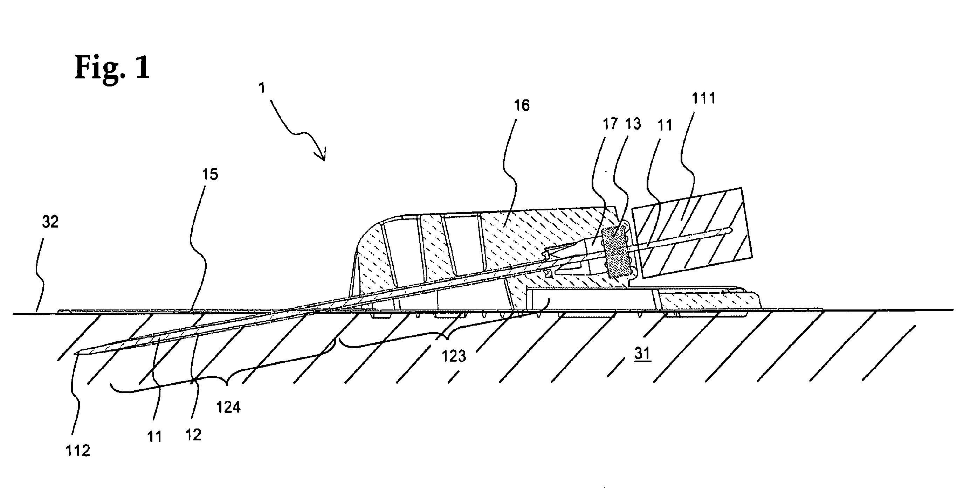

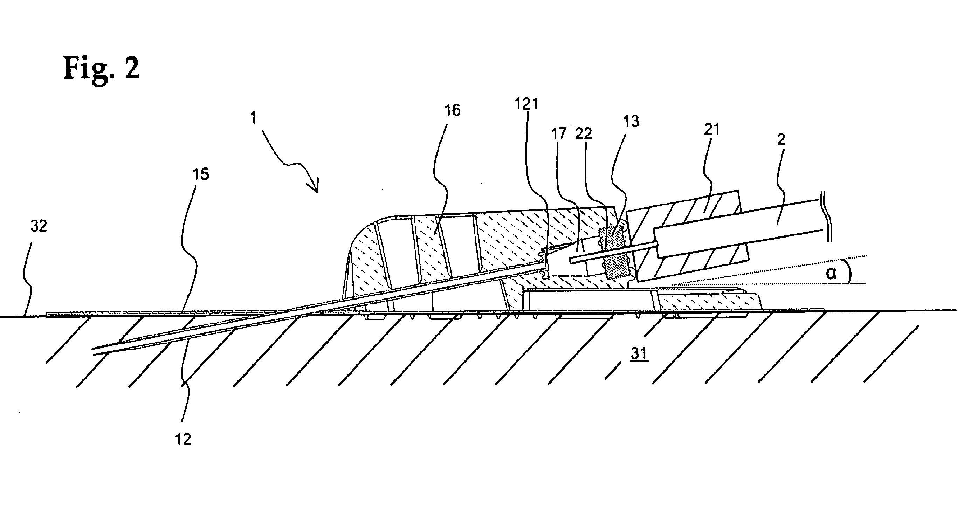

Figures 1 and 2 depict an example of a known infusion site interface 1 with angled infusion cannula 12, which is similar to an infusion site interface known from WO 02/07804 A1. Another similar infusion site interface is part of an infusion set distributed by Roche Diabetes Care AG, Burgdorf, Switzerland, under the name Accu-Check® TenderLink®.

Figure 1 shows a cross-section through the infusion site interface 1 attached to the body of a patient, directly after insertion of the infusion cannula. Figure 2 shows the infusion site interface 1 after the insertion needle 11 has been removed and the infusion tubing 2 has been connected to the interface 1.

The infusion site interface 1 comprises an interface body 16 mounted on an adhesive pad 15 for attaching the interface to the skin 32 of a patient An infusion cannula 12 is arranged in an acute angle to the pad 15. One part 123 of the cannula is mounted in the interface body 16, and the other part 124 projects to the adhesive side of the pad through an opening in the pad. A first end 121 of the cannula 12 opens toward a cavity 17 in the interface body 16, the cavity being sealingly closed by a septum 13. An insertion needle 11 is arranged in the cannula 12, and the septum 13. The needle tip 112 sticks out of the second end 122 of the cannula. On the other end the insertion needle 11 is provided with a grip 111.

For mounting the interface 1 the protective cap of the insertion needle (not shown) is removed, cannula 12 and needle 11 are inserted into the subcutaneous tissue 31, the cover foil (not shown) of the adhesive pad 15 is removed, and the pad 15 is adhered to the skin 32 of the patient, finally arriving at Figure 1. The user then removes the insertion needle 11 by grasping the needle grip 111 and retracting the needle 11 from the cannula 12 and the septum 13. The septum 13 sealingly closes the cavity 17. With the needle removed the infusion cannula 12 establishes a fluid connection between body tissue 31 and cavity 17.

In a next step the infusion tubing 2 of the infusion set is connected to the infusion site interface 1, as it is shown in Figure 2. One end of the infusion tubing is provided with a connector 21 that can be coupled (not shown in detail) to the infusion site interface. The connector 21 comprises a hollow needle 22. When the connector 21 is coupled to the interface 16, the hollow needle 22 penetrates the septum 13 and establishes a fluid connection between the infusion tubing 2 and the cavity 17.

As can be seen in Figure 2, the needle 22 of the connector 21 is collinear with the cannula 12, and is oriented at a certain angle α to the plane defined by the pad 15, or the skin respectively. As a result the connector 21 protrudes in regard to the interface body 16, leaving a gap between pad 15 and connector, which makes such an infusion site interface and the connector prone to being accidentally catched on clothing or the like. Thus there is the permanent risk of ripping the cannula out of the patient's body, or at least inadvertently disconnecting the infusion site interface from the infusion tubing.

Other types of infusion site interfaces are known that comprise two separate septums. One septum is used to sealingly close the cavity when the insertion needle is removed from the cannula, and a second septum is used to fluidly connect the cavity with an infusion tubing or a pump system via a hollow needle penetrating the second septum. Generally such arrangements are used for infusion site interfaces with cannulas that penetrate the skin perpendicularly. Thus the first septum is arranged collinear with the perpendicular insertion needle. The second septum is arranged in a right angle to the first septum, in order to allow attaching a connecting infusion tubing horizontally, parallel to the adhesive pad and the patient's skin.

The manufacture of infusion site interfaces with two septums is more expensive than with one septum, since the injection molding is more complicated. In addition such interfaces have a higher overall height, which again increases the risk of getting caught on clothes. Furthermore the volume of the cavity in the interface may have to be larger for geometrical reasons. Volumes that cannot be emptied (the so called dead volume), such as the volume of the cavity, are preferably as small as possible in infusion pump applications.

There is a need for infusion site interfaces that allow easy and safe use, have a low profile, and are less prone to accidentally catching on clothing, obstacles or the like.

### Objects of the Invention

It is an object of this invention to provide an infusion site interface that can be safely coupled to an infusion tubing, with a decreased risk of parts of the interface or a tubing connector getting caught on obstacles. Such an infusion site interface should have a small over-all height.

The infusion site interface should be producible at low costs. Advantageously it is compatible with existing types of connectors for infusion tubing.

These and other objects are achieved by an infusion site interface, an infusion set and an infusion pump system according to the independent claims. Advantageous embodiments are given in the dependent claims.

### Summary of the invention

An infusion site interface according to the invention comprises an interface body mounted on a pad adapted for being adhesively attached to a body of a patient; a soft infusion cannula adapted for being inserted into the body of the patient, wherein the cannula is connected to the interface body; a cavity arranged within the interface body, fluidly connected to the cannula; and one single septum that sealingly closes the cavity. The single septum is adapted to be penetrable by an insertion needle arranged in the infusion canula, in a first angle β-ε to its perpendicular, and is adapted to be penetrable by a hollow needle of an infusion tubing connector coupled to the infusion site interface, in a second angle ε to its perpendicular. The infusion site interface comprises guiding means that are adapted to guide said hollow needle of the connector during coupling, parallel to a plane defined by the pad. Advantageously such an interface device comprises an insertion needle arranged in the infusion cannula and penetrating the septum.

Surprisingly it was found that it is possible to use one single septum for both the insertion needle and the hollow needle of the connector, although the two needles are not collinear to each other. Thus, one single septum can be used for both needle. At the same time the hollow needle, and thus also the connector and the adjacent tubing, can be parallel to the plane defined by the pad of the interface, which allows to obtain a low profile of the interface with coupled connector.

An infusion set and an infusion pump system according to the invention comprise an infusion site interface according to the invention.

### Brief description of the drawings

In order to facilitate a fuller understanding of the present invention, reference is now made to the appended drawings. These references should not be construed as limiting the present invention, but are intended to be exemplary only.
- Figure 1: shows a cross-section through a prior art infusion site interface with angled infusion cannula, attached to the body of a patient, after insertion of the infusion cannula.
- Figure 2: shows a cross-section through a prior art infusion site interface with angled infusion cannula, attached to the body of a patient, after removal of the insertion needle and with established fluid connection to an infusion tubing.
- Figure 3: shows a cross-section through a infusion site interface according to the invention, after insertion of the infusion cannula.
- Figure 4: shows a cross-section through a infusion site interface according to the invention, after removal of the insertion needle and with established fluid connection to an infusion tubing.
- Figure 5: schematically shows the arrangement of the various axes in four possible variants of an infusion site interface according to the invention.

### Description of embodiments of the invention

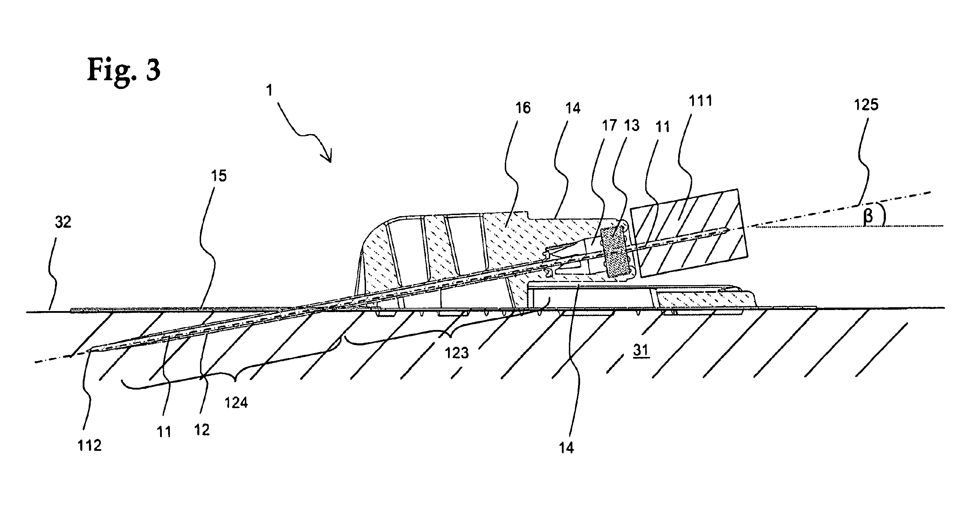

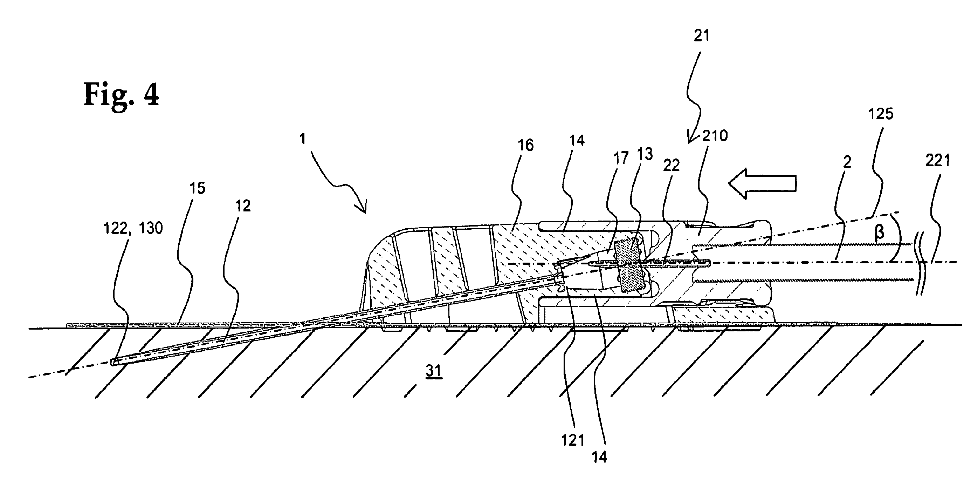

An advantageous embodiment of a device according to the invention is shown in Figures 3 and 4. Figure 3 shows a cross-section through the infusion site interface 1 attached to the body of a patient, directly after insertion of the infusion cannula, while Figure 4 shows the infusion site interface 1 after the insertion needle 11 has been removed and the infusion tubing 2 has been connected to the interface 1.

The shown infusion site interface according to the invention 1 comprises an interface body 16 mounted on an adhesive pad 15 for attaching the interface to the skin 32 of a patient.

An infusion cannula 12 is arranged at an acute angle β to the pad 15. The angle β is around 10 degrees, but this mainly depends on the particular design of the interface. One part 123 of the cannula is mounted in the interface body 16, and the other part 124 projects to the adhesive side of the pad through an opening in the pad. A first end 121 of the cannula 12 opens toward a cavity 17 in the interface body 16, the cavity being sealingly closed by a septum 13. An insertion needle 11 is arranged in the cannula 12, and the septum 13. The septum 13 is mounted in the interface body 16 such that its surface is perpendicular to the longitudinal axis 125 of the cannula 12.

An essential difference of the shown embodiment of an interface according to the invention in regard to the state of the art interface as shown in Figure 1 are the guiding means 14 provided on the interface body 16. Said guiding means 14 are adapted to guide a connector 21 of the infusion tubing 2 with its hollow needle 22 parallel to the plane defined by the pad 15.

As can be seen in Figure 4, a connector 21 adapted to be fluidly connected with the infusion site interface 1 according to the invention comprises a connector body 210 and guiding means 211. Said guiding means 211 can interact with the guiding means 14 of the interface body 16. The connector comprises a hollow needle 22 that is adapted to penetrate the septum 13 of the interface 1. The needle 22 is collinear with the tubing 2 adjacent to the connector body 210. When a user couples the connector 21 to the interface 1, the guiding means 14, interacting with the guiding means 211, guide the connector 21 and the hollow needle 22 parallel to the plane defined by the pad 15. As a result, the hollow needle 22 penetrates the septum 13 at angle β to its perpendicular 130.

Due to the advantageous guiding means 14, the coupled connector 21 is mounted horizontal on the interface body 16, with an essentially flat overall surface of interface body 16 and connector body 210. The tubing 2 adjacent to the connector leaves the connector parallel to the pad 15 and thus to the skin surface of the patient. Such an infusion site interface 1 according to the invention thus provides a low profile and smooth surface, with a considerably smaller risk of getting caught on clothing or the like.

It is even possible to apply adhesive tape or an additional adhesive pad to cover a part of or even the complete infusion site interface with coupled connector, in order to achieve a smooth surface. This further decreases the risk of ripping the cannula out of the patient's body, or inadvertently disconnecting the infusion site interface from the infusion tubing. Yet another advantage of such an additional cover is the possibility to camouflage the complete infusion site interface, by using a skin colored adhesive pad.

On the other hand, covering a prior art infusion site interface with coupled connector as shown in Figure 2 with tape would be rather unadvisable, since the connector would be subject to shearing forces that could detach the interface from the skin, and could destroy the connection.

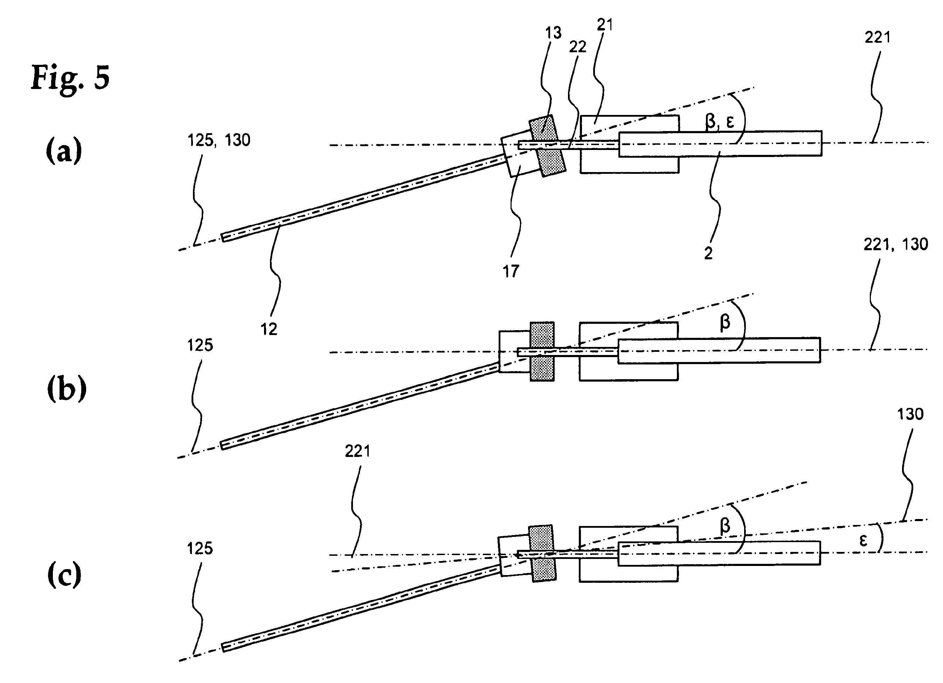

The embodiment shown in Figures 3 and 4 represents one possible arrangement of the cannula axis, the connector needle axis, and the axis of the septum. Other possible layouts of an infusion site interface according to the invention are shown in Figures 5(a) to (d). For a better understanding only the components necessary for explaining the different embodiments are shown.

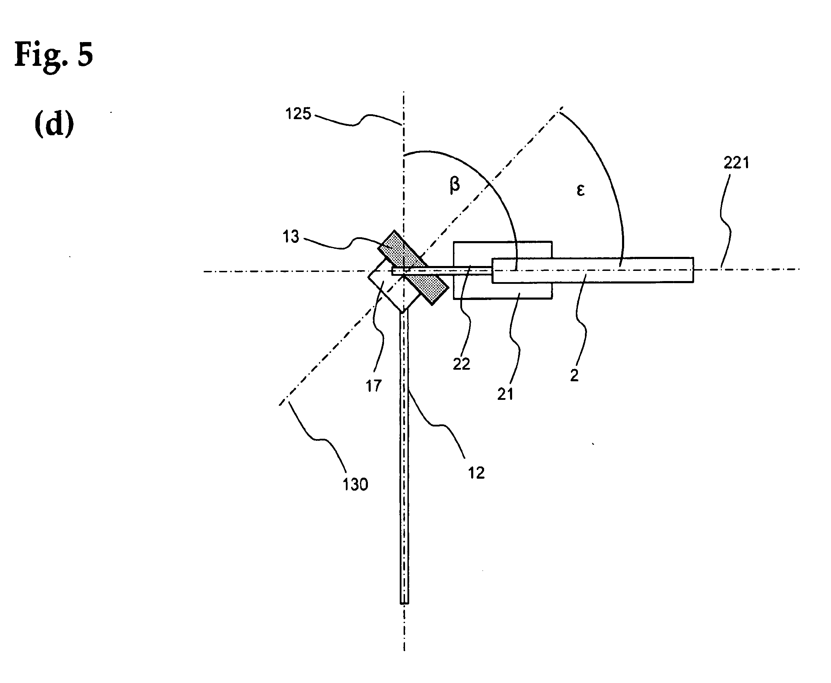

Figure 5(a) schematically shows the embodiment shown in Figures 3 and 4. As in all schemes in Figure 5, the axis 221 of the hollow needle 22 of the connector 21 is arranged parallel to the plane defined by the adhesive pad (not shown) of the interface according to the invention. An infusion cannula 12 is arranged with its axis 125 at an angle β to axis 221. The axis 130 of the septum 13 is parallel to the cannula axis 125. The angle between axis 130 and pad plane/axis 221 is denominated ε.

Figure 5(b) shows another embodiment, in which the axis 130 of the septum is parallel to the hollow needle axis 221, and thus angle ε is zero degrees. In contrast to the embodiment in Figure 5(a) the insertion needle (not shown) arranged in the cannula 12 penetrates the septum surface at an angle β, while the hollow needle 22 penetrates the septum perpendicularly (ε = 0).

A further variant is shown in Figure 5(c), where the angle ε is chosen smaller than the angle β. As a result, both the insertion needle (not shown) as well as the hollow needle penetrate the septum at an angle. This tilted arrangement of the septum in regard to both axes 125 and 221 has the advantage that the maximum deviation from the septum axis 130 is smaller than in Figures 5(a) and (b).

Figure 5(d) discloses an embodiment in which such an arrangement is particularly advantageous. The cannula 12 is arranged perpendicularly to the pad of the device, and thus will be perpendicular to the skin surface (β = 90 degrees). On the other hand the hollow needle 22 of the connector is still parallel to the plane defined by the pad (not shown). The septum 13 is arranged such that its axis 130 has an angle ε = 45 degrees. Thus both the insertion needle and the hollow needle 22 will penetrate the septum 13 at an angle of 45 degrees to its perpendicular 130.

### List of Reference Numerals

- 1: infusion site interface
- 11: insertion needle
- 111: grip
- 112: needle tip
- 12: infusion cannula
- 121: first end of infusion cannula
- 122: second end of infusion cannula
- 123: first part of infusion cannula
- 124: second part of infusion cannula
- 125: axis of cannula
- 13: septum
- 130: axis of septum, perpendicular of septum surface
- 14: guiding means of interface
- 15: carrier, adhesive pad
- 16: interface body
- 17: cavity
- 2: infusion tubing
- 21: tubing connector
- 210: connector body
- 211: guiding means of connector
- 22: hollow needle
- 221: axis of hollow needle
- 31: subcutaneous body tissue
- 32: skin

## Claims

1. An infusion site interface (1) comprising an interface body (16) mounted on a pad (15) adapted for being adhesively attached to a body of a patient; a soft infusion cannula (12) adapted for being inserted into the body of the patient, wherein the cannula (12) is connected to the interface body (16); a cavity (17) arranged within the interface body (16), fluidly connected to the cannula (12); and one single septum (13) that sealingly closes the cavity (17), **characterized in that** the single septum (13) is adapted to be penetrable by an insertion needle (11) arranged in the infusion canula (12), in a first angle (β-ε) to its perpendicular (130), and is adapted to be penetrable by a hollow needle (22) of an infusion tubing connector (21) coupled to the infusion site interface (1), in a second angle (ε) to its perpendicular (130); wherein the infusion site interface (1) comprises guiding means (14) that are adapted to guide said hollow needle (22) of the connector (21) during coupling, parallel to a plane defined by the pad (15).

2. The infusion site interface according to claim 1, **characterized by** an insertion needle (11) arranged in the infusion cannula (12) and penetrating the septum (13).

3. An infusion set with an infusion site interface (1) according to claim 1 or 2.

4. An infusion pump system with an infusion site interface (1) according to claim 1 or 2.

## Patentansprüche

1. Infusionsstellenschnittstelle (1), umfassend einen Schnittstellenkörper (16), der auf einem Pad (15) montiert ist, das dazu ausgelegt ist, haftend auf einem Körper eines Patienten befestigt zu werden; eine weiche Infusionskanüle (12), die dazu ausgelegt ist, in den Körper des Patienten eingeführt zu werden, wobei die Kanüle (12) mit dem Schnittstellenkörper (16) verbunden ist; einen im Schnittstellenkörper (16) angeordneten Hohlraum (17), der fluidisch mit der Kanüle (12) verbunden ist; und ein einzelnes Septum (13), das den Hohlraum (17) dichtend abschliesst, **dadurch gekennzeichnet, dass** das einzelne Septum (13) dazu ausgelegt ist, durch eine in der Infusionskanüle (12) angeordnete Einführungsnadel (11) durchdringbar zu sein, in einem ersten Winkel (β-ε) zu seiner Senkrechten (130), und dazu ausgelegt ist, durch eine an die Infusionsstellenschnittstelle (1) gekoppelte Hohlnadel (22) eines Infusionsschlauchverbinders (21) durchdringbar zu sein, in einem zweiten Winkel (ε) zu seiner Senkrechten (130); wobei die Infusionsstellenschnittstelle (1) Führungsmittel (14) umfasst, die dazu ausgelegt sind, die genannte Hohlnadel (22) des Verbinders (21) während der Kopplung zu führen, parallel zur Ebene, die durch das Pad (15) definiert ist.

2. Infusionsstellenschnittstelle nach Anspruch 1, **gekennzeichnet durch** eine Einführungsnadel (11), die in der Infusionskanüle (12) angeordnet ist und das Septum (13) durchdringt.

3. Infusionsset mit einer Infusionsstellenschnittstelle (1) nach Anspruch 1 oder 2.

4. Infusionspumpensystem mit einer Infusionsstellenschnittstelle (1) nach Anspruch 1 oder 2.

## Revendications

1. Interface de site de perfusion (1) comprenant un corps d'interface (16) monté sur une compresse (15) agencée pour être fixée par adhésif à un corps d'un patient; une canule de perfusion souple (12) agencée pour être introduite dans le corps du patient, la canule (12) étant reliée au corps d'interface (16); une cavité (17) prévue à l'intérieur du corps d'interface (16), reliée fluidiquement à la canule (12); et un unique septum (13) qui ferme de manière étanche la cavité (17), **caractérisée par le fait que** l'unique septum (13) est agencée pour être apte à être pénétrée par une aiguille d'introduction (11) disposée dans la canule de perfusion (12), dans un premier angle (β-ε) par rapport à sa perpendiculaire (130), et est agencée pour être apte à être pénétrée par une aiguille creuse (22) d'un raccord de tubulure de perfusion (21) couplé à l'interface de site de perfusion (1), dans un second angle (ε) par rapport à sa perpendiculaire (130); l'interface de site de perfusion (1) comprenant des moyens de guidage (14) qui sont agencés pour guider ladite aiguille creuse (22) du raccord (21) durant un couplage, parallèlement à un plan défini par la compresse (15).

2. Interface de site de perfusion selon la revendication 1, **caractérisée par** une aiguille d'introduction (11) disposée dans la canule de perfusion (12) et pénétrant le septum (13).

3. Ensemble de perfusion ayant une interface de site de perfusion (1) selon l'une des revendications 1 ou 2.

4. Système de pompe à perfusion ayant une interface de site de perfusion (1) selon l'une des revendications 1 ou 2.
